Europäisches Patentamt

European Patent Office

Office européen des brevets

(19)

(11) **EP 0 546 916 B1**

(12) **FASCICULE DE BREVET EUROPEEN**

(45) Date de publication et mention
de la délivrance du brevet:
**14.05.2003 Bulletin 2003/20**

(51) Int Cl.⁷: **G01N 33/92**, G01N 27/26

(21) Numéro de dépôt: **92403309.5**

(22) Date de dépôt: **07.12.1992**

(54) **Procédé de séparation de la Lp(a) par électrophorèse et application à la détermination in vitro du risque athérogène lié à la présence de la Lp(a)**

Verfahren zur Lp(a)-Trennung durch Elektrophorese und Anwendung des Verfahrens auf die in vitro Bestimmung des Atherogen-Risikos in Zusammenhang mit der Anwesenheit des Lp(a)

Method for the separation of Lp(a) by electrophoresis and its application to the in vitro determination of atherogenic risk linked to the presence of Lp(a)

(84) Etats contractants désignés:
**AT BE CH DE DK ES FR GB GR IE IT LI LU MC NL PT SE**

(30) Priorité: **11.12.1991 FR 9115387**

(43) Date de publication de la demande:
**16.06.1993 Bulletin 1993/24**

(73) Titulaire: **SEBIA**
**92130 Issy-Les-Moulineaux (FR)**

(72) Inventeurs:
• **Bellon, Franck**
  **F-91160 Longjumeau (FR)**
• **Bringard, Aline**
  **F-91090 Lisses (FR)**

(74) Mandataire: **Desaix, Anne et al**
**Ernest Gutmann - Yves Plasseraud S.A.**
**3, rue Chauveau-Lagarde**
**75008 Paris (FR)**

(56) Documents cités:
**WO-A-82/02599        WO-A-89/00689**

• **AMERICAN JOURNAL OF HUMAN GENETICS vol. 49, no. 5, Novembre 1991, CHICAGO,USA pages 1063 - 1074 KAMBOH M.I. ET AL. 'Expressed hypervariable polymorphism of apolipoprotein(a)'**

• **ELECTROPHORESIS vol. 7, no. 5, Mai 1986, WEINHEIM,GERMANY pages 197 - 203 BOERSUM T. ET AL. 'Electrophoretic migration velocity of amphiphilic proteins increases with decreasing Triton X-100 concentration: A new characteristic for their identification'**

• **JOURNAL OF BIOLOGICAL CHEMISTRY. vol. 257, no. 1, 10 Janvier 1982, BALTIMORE US pages 501 - 507 G.UTERMANN ET AL. 'Genetic variants of group A apolipoproteins'**

• **CLINICA CHIMICA ACTA vol. 188, no. 1, 1990, AMSTERDAM,NL pages 71 - 77 BRUCKERT E. ET AL. 'Does electrophoresis reliably screen for high serum lipoprotein(a)?'**

• **JOHANSSON B.G.: 'Agarose gel electrophoresis' SCAND. JOURN. CLIN. LAB. INVEST. vol. 29, 1972, pages 7 - 19**

• **NOBLE R.P.: 'Electrophoretic separation of plasma lipoproteins in agarose gel' JOURN. LIPID RES. vol. 9, no. 6, Novembre 1968, pages 693 - 700**

Remarques:
Le dossier contient des informations techniques présentées postérieurement au dépôt de la demande et ne figurant pas dans le présent fascicule.

**Description**

La présente invention a pour objet un procédé de séparation de la Lp(a) par électrophorèse, des gels pour la mise en oeuvre de ce procédé, ainsi que l'application de ce procédé à la détermination du risque athérogène lié à la présence de la Lp(a) et plus particulièrement des risques que présente un individu d'être atteint d'une maladie telle que l'athérosclérose ou toute autre pathologie liée à cette dernière.

L'électrophorèse du sérum ou du plasma humain en gel d'agarose, suivie d'une coloration spécifique des lipides, permet de séparer et de visualiser les différentes fractions lipoprotéiques, HDL, VLDL, LDL et chylomicrons, dont les taux respectifs sont de précieux indicateurs des risques d'athérosclérose (FREDRICKSON et al., 1967, New Engl. J. Med., 276).

La "Lp(a)" est une lipoprotéine particulière, identifiée par BERG en 1963 (Acta Pathol. Microbiol. Scand., 59, 369). Elle présente une grande similitude structurale avec les LDL, mais est caractérisée par une partie protéique supplémentaire, appelée apo(a), liée à l'apo B100 par des ponts disulfures.

Bien que le rôle physiologique de la Lp(a) ne soit pas encore parfaitement élucidé, de nombreuses études cliniques ont montré qu'un taux élevé de Lp(a) plasmatique (supérieur à 0,3 g/l) constitue un facteur indépendant de risque athérogène (KOSTNER et al., 1981, Atherosclerosis, 38, 51; RHOADS et al. 1986, J. Ann. Med. Assoc., 256, 2540).

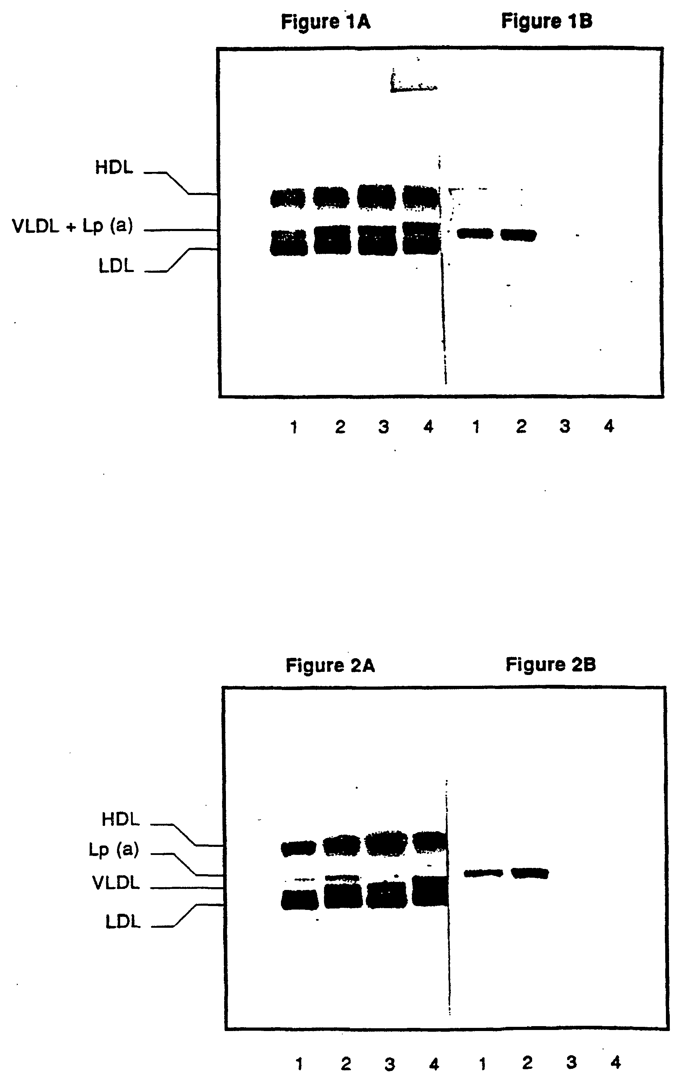

Actuellement, l'électrophorèse classique du sérum ou du plasma humain (gel standard : agarose 0,5 %, en tampon Tris Véronal; voir Figure 1A), permet généralement la résolution de trois fractions lipoprotéiques: les HDL (position α), les VLDL (position préβ) et les LDL (position β). La fraction Lp(a), quand elle est présente, migre en position préß1, et ne peut généralement pas être distinguée des VLDL de manière suffisante pour conclure de façon certaine sur sa présence ou son absence dans le cadre de la détermination du risque athérogène lié à la présence de la Lp(a) (BRUCKERT et al., 1990, Clin. Chim. Acta, 188, 71).

La demande de brevet WO 89/00689 fait référence à une publication de Johansson B.G. (Scand. J. Clin. Lab. Invest., 29, Suppl. 124, 7-19, 1972)qui décrit une technique d'électrophorèse permettant de séparer et d'identifier plus de composants que les 4 à 7 fractions habituelles. Selon Johansson B.G., des conditions spécifiques d'électrophorèse et l'addition d'ions calcium au tampon Barbital permettent de visualiser les lipoprotéines α, β, et préβ.

Par conséquent, les différentes méthodes permettant de dépister ou de doser la Lp(a) plasmatique reposent toutes, à l'heure actuelle, sur l'utilisation d'anticorps spécifiques. Ces méthodes sont plus particulièrement les suivantes: l'immunonéphélémétrie (CAZZOLATO et al., 1983, Clin. Chim. Acta, 135, 203), l'électrosynérèse (MOLINARI et al., 1983, Clin. Chim. Acta, 128, 373), l'électroimmunodiffusion selon LAURELL (KOSTNER et al., 1981, Atherosclerosis, 38, 51), les techniques immunoenzymatiques ELISA (ABE et al., 1988, Clin. Chim. Acta, 177, 31), ainsi qu'une technique d'immunolatex (VU DAC et al., 1985, J. Lipid. Res., 26, 267).

Cependant, ces dosages sont généralement coûteux, et ne sont pas prescrits systématiquement par le clinicien dans le cadre d'une recherche de dyslipoprotéinémie. Préalablement à un tel dosage, il serait donc très intéressant de pouvoir dépister la présence de Lp(a) en quantité pathologique lors d'une analyse lipoprotéique courante.

La publication CLINICA CHIMICA ACTA, vol. 188, N° 1, 1990 décrit une technique pour la mise en évidence de la lipoprotéine (a) contenue dans un sérum, par électrophorèse sur un gel d'agarose.
Selon cette publication, lorsque la Lpa est présente, elle est située entre les fractions LDL et VLDL.

Le document THE AMERICAN JOURNAL OF HUMAN GENETICS, vol. 49, N° 5 (Novembre 1991) décrit l'étude des phénotypes de l'apolipoprotéine A (APO(a)) et décrit à cet égard l'utilisation d'un gel d'agarose SDS suivie d'un transfert sur nitrocellulose pour réaliser un immunoblot avec des anticorps spécifiques anti-APO(a). Préalablement à cette étude, en vue de déterminer le polymorphisme de APO(a), les auteurs séparent les sous-unités APO(a) et APO (b) de la lipoprotéine A (Lp(a)).
Une publication ELECTROPHORESIS, vol. 7, N° 5, Mai 1986 décrit des tests de migration électrophorétique réalisés sur des protéines hydrophiles et amphiphiles, en présence de Triton X-100. Les auteurs de cet article ont étudié l'influence de la présence de Triton X-100 sur la vitesse de migration de ces protéines.
Une publication de JOURNAL OF BIOLOGICAL CHEMISTRY, vol. 257, N° 1, 1982 décrit la séparation des apolipoprotéines hydrophobes par rapport aux protéines hydrophiles, au moyen d'une électrophorèse sur gel d'agarose en présence de détergent. Ensuite les apolipoprotéines délipidées sont analysées par électrophorèse.

Un des buts de la présente invention est précisément de mettre à la disposition des cliniciens, un procédé de séparation de la Lp(a) des autres lipoprotéines du sérum, qui soit d'un prix de revient nettement inférieur aux procédés actuels, et qui, par conséquent, puisse être prescrit dans le cadre d'une recherche de dyslipoprotéinémie courante.

La présente invention a également pour but de permettre le dépistage de façon fiable de la Lp(a), et par conséquent le diagnostic in vitro fiable du risque d'apparition chez un individu de pathologies liées à l'athérosclérose.

L'invention concerne un procédé de séparation par électrophorèse de la Lp(a) et des différentes lipoprotéines contenues dans un milieu biologique, caractérisé en ce que la migration électrophorétique des lipoprotéines est réalisée dans des conditions telles que le gel d'électrophorèse et/ou le milieu biologique sus-mentionné contient des composés susceptibles de modifier de façon différentielle la mobilité électrophorétique de la Lp(a), par rapport a celle des autres lipoprotéines, ces composés étant choisis parmi les molécules ayant une partie hydrophobe et chargées négativement, les agents tensio-actifs neutres, ou les cations ayant un degré d'oxydation supérieur ou égal à 2, ou les complexes cationiques choisis parmi $NH_4^+$, $(NH_4^+)_2Fe^{2+}$, $NH_4^+Fe^{3+}$.

Le procédé sus-mentionné est plus particulièrement caractérisé en ce qu'il permet de séparer systématiquement, dans un milieu biologique, la fraction Lp(a) des fractions VLDL et LDL, de préférence tout en conservant la séparation des HDL, LDL et VLDL.

Selon un mode de réalisation particulièrement

avantageux du procédé de l'invention, les composés susceptibles de modifier de façon différentielle la mobilité électrophorétique de la Lp(a), par rapport à celle des autres lipoprotéines, sont incorporés dans le gel d'électrophorèse utilisé. C'est avantageusement le cas des cations.

Selon un autre mode de réalisation particulièrement avantageux du procédé de l'invention, les composés susceptibles de modifier de façon différentielle la mobilité électrophorétique de la Lp(a), par rapport à celle des autres lipoprotéines, et plus particulièrement les molécules ayant une partie hydrophobe et chargées négativement ou les agents tensio-actifs neutres, sont introduits dans le milieu biologique avant de procéder à l'électrophorèse sur gel classique.

De préférence, les molécules ayant une partie hydrophobe et chargées négativement susceptibles d'être additionnées au milieu biologique, sont plus particulièrement celles caractérisées en ce qu'elles ont des propriétés tensio-actives.

Selon un autre mode de réalisation de l'invention, les gels de l'invention peuvent également être préparés à partir de gels classiques modifiés par pénétration des composés chargés, anioniques ou cationiques, préalablement incorporés dans le tampon de migration. Cette incorporation est réalisée soit au cours d'une prémigration électrophorétique précédant le dépôt des échantillons (le dépôt ayant lieu soit dans la zone du gel où ces composés chargés auront pénétré, soit à proximité de cette zone) soit au cours de l'électrophorèse proprement dite.

Les composés chargés, anioniques ou cationiques, peuvent indifféremment être incorporés aux tampons anodiques ou cathodiques ou bien pour les composés anioniques uniquement au tampon cathodique et pour les composés cationiques uniquement au tampon anodique.

Lorsque le dépôt de l'échantillon (milieu biologique) est effectué dans une zone d'un gel dans lequel les composés cationiques ou anioniques ont pénétré par une étape de préélectrophorèse, le lipidogramme obtenu est identique à celui qui serait obtenu avec un gel dans lequel ces composés cationiques ou anioniques auraient été introduits directement lors du coulage du gel.

En revanche, si le dépôt des échantillons est réalisé dans une zone de gel non encore atteinte par ces composés cationiques ou anioniques, étant entendu que les lipoprotéines de l'échantillon seront amenées au contact de ces composés cationiques ou anioniques lors de la migration électrophorétique proprement dite de ces échantillons, le profil obtenu sur le lipidogramme sera la résultante de la séparation obtenue sur un gel classique et de la séparation obtenue sur un gel modifié selon l'invention. Il sera d'autant plus semblable au lipidogramme qui serait obtenu sur un gel où les composés cationiques ou anioniques sont présents, que le dépôt de l'échantillon effectué après l'étape de préélectrophorèse sera réalisé à plus faible distance de la zone du gel où ces composés cationiques ou anioniques auront pénétré par la prémigration.

L'addition de ces composés dans le gel ou le milieu biologique modifie de façon différentielle la mobilité électrophorétique de la Lp(a) par rapport à celle des autres lipoprotéines.

Les cations ou complexes cationiques ou les agents tensio-actifs neutres présentent la propriété de ralentir la vitesse de migration électrophorétique des lipoprotéines par rapport à la vitesse de migration de ces mêmes lipoprotéines sur un gel d'électrophorèse classique ne contenant pas ces cations ou complexes cationiques ou ces agents tensio-actifs neutres. Cependant, la vitesse de migration de la Lp(a) est moins ralentie que celle des autres lipoprotéines, ou est même non modifiée par rapport à celle obtenue avec un gel ne contenant pas de cations ou de complexes cationiques ou d'agents tensio-actifs neutres.

Pour une lipoprotéine donnée, si l'on considère que $d/d_T$ représente le rapport entre la distance de migration sur le gel contenant le composé et la distance de migration sur le gel témoin de cette même lipoprotéine, et que la fraction Lp(a) est individualisée si sa distance par rapport à la fraction la plus proche (à savoir la VLDL) est d'au moins 3 mm, le facteur de freinage ($d/d_T$) de la fraction VLDL caractérisant les procédés de l'invention utilisant des cations ou complexes cationiques ou des agents tensio-actifs neutres, est inférieur ou égal à 0,7.

Les molécules ayant .une partie hydrophobe et chargées négativement présentent quant à elles la propriété d'accélérer la vitesse de migration électrophorètique des lipoprotéines par rapport à la vitesse de migration de ces mêmes lipoprotéines sur un gel d'électrophorèse classique ne contenant pas ces molécules chargées négativement. Quant à la Lp(a), sa vitesse de migration n'est sensiblement pas modifiée par la présence d'une molécule sus-mentionnée.

A ce titre, toujours dans l'hypothèse où la fraction Lp(a) est individualisée si sa distance par rapport à la fraction la plus proche (à savoir la LDL) est d'au moins 3 mm, le facteur d'accélération (d/d$_T$) de la fraction LDL caractérisant les procédés de l'invention utilisant des molécules ayant une partie hydrophobe et chargées négativement, est au moins de l'ordre de 2,3.

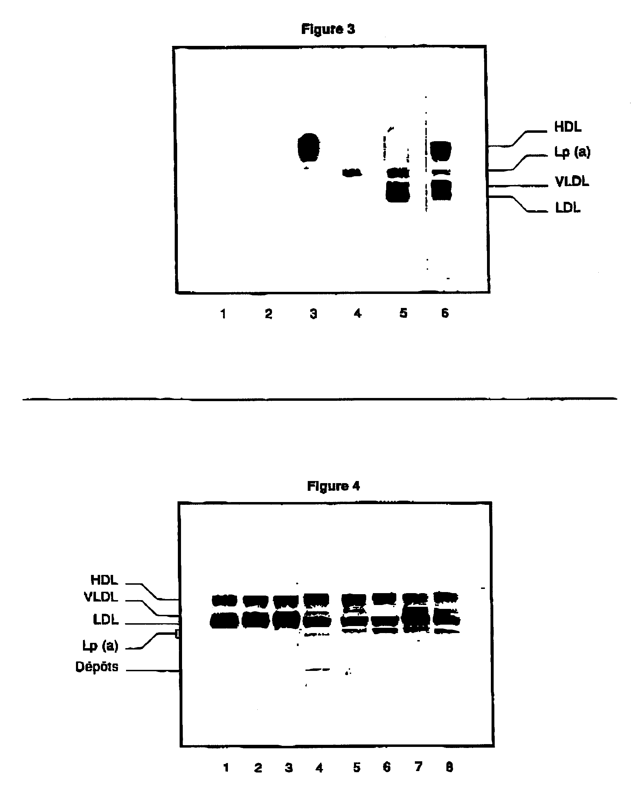

Comme vu précédemment, la vitesse de migration des lipoprotéines sur le gel est diminuée en présence de cations ou complexes cationiques, la Lp(a) étant moins ralentie que les autres lipoprotéines, elle devient dans ces conditions nettement individualisée sur le gel. Sur le lipidogramme de la Figure 2A, la présence d'une fraction supplémentaire entre les VLDL et les HDL caractérise donc la Lp(a). Cette fraction supplémentaire a été identifiée sans ambiguïté par immunofixation : la bande lipoprotéique en question est révélée par les anticorps spécifiques dirigés contre les apolipoprotéines apoB et apo(a), alors qu'elle ne réagit pas avec les anti-apo-AI, -apo-AII -apo-CIII et -apo-E (Figure 3).

Un procédé particulièrement avantageux dans le cadre de la présente invention est caractérisé en ce que le composé susceptible de modifier de façon différentielle la mobilité électrophorétique de la Lp(a) par rapport à celle des autres lipoprotéines est choisi parmi les cations ou complexes cationiques dont les hydroxydes, à pH allant d'environ 8 à environ 9, ne sont pas précipités ou sont partiellement précipités, de telle sorte qu'il y ait au moins $10^{-3}$ moles/l de cations ou de complexes cationiques non précipités dans le gel pour électrophorèse.

De façon avantageuse, les cations sont choisis parmi les colonnes IIa, IIIa et IIIb du tableau de Mandeleiev.

Ces cations sont avantageusement choisis parmi $Mg^{2+}$, $Ca^{2+}$, $Ba^{2+}$, $Sr^{2+}$, $Mn^{2+}$, $Be^{2+}$, $Al^{3+}$, $La^{3+}$, $Ce^{3+}$, $In^{3+}$ et $Y^{3+}$.

De préférence, le cation utilisé est $Mg^{2+}$.

La concentration des cations ou complexes cationiques dans le gel est avantageusement de l'ordre de $10^{-3}$ à $10^{-1}$ M, et de préférence de l'ordre de $5.10^{-3}$ à $3.10^{-2}$ M. L'effet observé de séparation de la Lp(a) et des autres lipoprotéines s'accentue en augmentant la concentration des cations ou complexes cationiques. Toutefois, l'incorporation d'une trop grande quantité de sel (au-delà de $10^{-1}$ M, voir même 0,06 M) n'est pas souhaitable, car ceci augmente le temps de migration nécessaire, et nuit à la séparation des fractions VLDL et LDL.

L'anion composant le sel en solution de ces cations ou complexes cationiques n'intervient pas dans l'effet observé de séparation de la Lp(a) et des autres lipoprotéines (chlorure, acétate, sulfate...).

Un autre procédé particulièrement préféré de l'invention est caractérisé en ce que le composé susceptible de modifier de façon différentielle la mobilité électrophorétique de la Lp(a) par rapport aux autres lipoprotéines est une molécule ayant une partie hydrophobe et chargée négativement.

De préférence, la molécule ayant une partie hydrophobe et chargée négativement,

. soit fait partie de la classe des agents tensio-actifs anioniques et comprend

* soit une chaîne aliphatique linéaire ou ramifiée de 3 à 10 atomes de carbone, comportant éventuellement des hétéroatomes, comportant une fonction acide telle que sulfurique, sulfonique, carboxylique ou phosphorique, et possédant une chaîne aliphatique condensée comportant au moins 1 à 6 cycles saturés de 5 ou 6 atomes de carbone,
* soit une chaîne aliphatique linéaire ou ramifiée d'au moins 8 atomes de carbone, de préférence de 10 à 18 atomes de carbone, cette chaîne étant le cas échéant substituée par un (ou plusieurs) cycle(s) ou hétérocycle(s) aromatique(s), ladite chaîne ou ce (ou ces) cycle(s) ou hétérocycle(s) aromatique(s) étant substitué(s) par une fonction acide telle que carboxylique, sulfonique ou phosphorique,

. soit est un dérivé aromatique chargé négativement et comportant au moins deux cycles aromatiques condensés tel que le naphtalène, ou un cycle aromatique condensé à un hétérocycle aromatique telle que la quinoléine, ou deux hétérocycles aromatiques condensés, l'un au moins des cycles comportant une fonction acide telle que sulfurique, carboxylique, sulfonique ou phosphorique etc... et l'autre cycle ne comportant pas de fonction susceptible de faire perdre à l'ensemble de la molécule son caractère hydrophobe et notamment ne comportant pas de charge polaire.

Parmi les agents tensio-actifs anioniques, on peut citer le sodium dodécylsulfate, le sodium dodécylbenzènesulfonate et le cholate de sodium.

Des dérivés aromatiques chargés négativement sont avantageusement choisis parmi les composés suivants:

- acide naphtalène carboxylique-2,
- acide naphtalène sulfonique-2,
- acide amino-2 naphtalène sulfonique-1,
- acide hydroxy-4 naphtalène sulfonique-1,

- acide amino-1 naphtalène sulfonique-5,
- Noir Amido,
- Violet Acide 17,
- Bleu de Coomassie R250,
- Palatine fast black wan,

et de préférence:

- acide hydroxy-1 naphtalène carboxylique-2,
- acide hydroxy-3 naphtalène carboxylique-2.

Pour les agents tensio-actifs anioniques, la gamme de concentration efficace est avantageusement dans le gel d'environ $10^{-6}$ à environ $10^{-3}$ M, et de préférence d'environ $10^{-4}$ à environ $10^{-3}$ M. Dans le cas où les agents tensio-actifs anioniques sont introduits dans le milieu biologique avant électrophorèse, leur concentration est avantageusement d'environ $10^{-4}$ M à $10^{-1}$ M, et de préférence d'environ $10^{-3}$ M à environ $10^{-2}$ M. Cette concentration est telle que la vitesse de la migration de la LDL est davantage accélérée que celle de la fraction Lp(a) de sorte que la distance minimale entre la Lp(a) et la fraction LDL, sur le gel, est d'au moins 3 mm (la Lp(a) se situant "derrière" la fraction LDL).

En ce qui concerne les dérivés aromatiques chargés négativement, la gamme de concentration efficace dans le gel est d'environ $10^{-3}$ M à environ $10^{-1}$ M, de préférence d'environ $10^{-2}$ M à environ $5.10^{-2}$ M, et s'ils sont introduits préalablement dans le milieu biologique leur gamme de concentration efficace est d'environ $10^{-2}$ M à environ 1 M, de préférence d'environ $5.10^{-2}$ M à $5.10^{-1}$ M.

Selon un autre mode de réalisation préférée de l'invention, le composé susceptible de modifier de façon différentielle la mobilité électrophorétique de la Lp(a) par rapport aux autres lipoprotéines est un agent tensio-actif neutre (ou détergent neutre).

De préférence l'agent tensio-actif neutre est tel qu'il est soluble en phase aqueuse, et est avantageusement choisi parmi le Triton X100®, Triton X405®, le Tween 20®, le NP40® et le BRIJ35®.

Lorsqu'ils sont dans le gel, les agents tensio-actifs neutres sont avantageusement à une concentration d'environ $10^{-6}$ à $10^{-3}$ M, de préférence de $10^{-4}$ M à $10^{-3}$ M. Lorsqu'ils sont introduits dans l'échantillon biologique, préalablement à l'électrophorèse, les agents tensio-actifs neutres sont avantageusement à une concentration d'environ $10^{-4}$ M à $10^{-1}$ M, et de préférence $10^{-3}$ M à $10^{-2}$ M.

Selon un autre mode de réalisation de l'invention, la séparation de la Lp(a) et des différentes lipoprotéines contenues dans un milieu biologique, est avantageusement réalisée sur un gel d'électrophorèse modifié par rapport à un gel classique, par un mélange de composés cationiques et de détergent neutre. Ces composés susceptibles de modifier la mobilité des Lp(a) sont ajoutés selon les modalités précédemment décrites conformément aux différents modes de réalisation de l'invention.

Les proportions respectives des composés cationiques et des détergents neutres utilisés sont déterminées par les propriétés de ces constituants, à savoir leur capacité à ralentir préférentiellement certaines fractions lipoprotéiques. Un ralentissement suffisant des VLDL permettant de visualiser la Lp(a) peut être obtenu en utilisant exclusivement un composé cationique ou un détergent neutre.

Mais dans le cas de l'utilisation d'un sérum très frais (ayant moins de 24h lorsqu'il est conservé à 4°C) la grande mobilité des VLDL observée oblige à utiliser une concentration très importante de composés cationiques et dans ce cas les LDL trop ralenties restent au point de dépôt et la force ionique atteinte est excessive ce qui entraine un temps de migration plus long. Dans ce cas on peut avantageusement ajouter un détergent neutre aux composés cationiques.

De même lorsqu'avec un sérum très frais on utilise seulement un détergent neutre, on peut observer un trop fort ralentissement de la fraction LDL qui peut masquer la Lp(a), ainsi qu'une distorsion des bandes. L'utilisation conjuguée, en proportions optimales déterminables par l'homme du métier, de composés cationiques et de détergent neutre permet d'obtenir le profil lipoprotéique souhaité.

Des gels pour électrophorèse appropriés pour réaliser l'invention comprenent des cations ou complexes cationiques.

Les cations ou complexes cationiques compris dans ces gels sont avantageusement choisis parmi ceux décrits ci-dessus.

Un autre gel pour électrophorèse utilisable pour réaliser l'invention comprend des molécules ayant une partie hydrophobe et chargées négativement, notamment celles choisies parmi les molécules décrites ci-dessus.

Un autre gel pour électrophorèse approprié comprend des agents tensio-actifs neutres, notamment ceux choisis parmi les agents décrits ci-dessus.

Un gel d'électrophorèse, également utilisable comprend en mélange, des composés cationiques et des détergents neutres.

L'invention vise plus particulièrement l'utilisation de ces gels pour la mise en oeuvre d'un procédé de séparation par électrophorèse de la Lp(a) et des différentes fractions lipoprotéiques contenues dans un milieu biologique, tel que décrit ci-dessus.

Un gel particulièrement préféré utilisé selon l'invention est celui comprenant à titre de cation $Mg^{2+}$, dans les conditions de concentration précisées ci-dessus.

Les gels utilisés selon l'invention sont, la présence de composés susceptibles de modifier de façon différentielle la mobilité électrophorétique de la Lp(a) par rapport aux autres lipoprotéines mise à part, des gels classiquement utilisés dans le domaine de l'électrophorèse, notamment des gels d'agarose, de polyacrylamide ou d'acétate de cellulose.

Les gels utilisés selon l'invention peuvent être préparés par mélange de la solution des composés sus-mentionnés avec celle du gel à chaud, suivi d'une étape de solidification du gel par retour à température ambiante.

Les gels utilisé selon l'invention peuvent également être préparés par immersion d'un gel dans une solution de composés sus-mentionnés, pendant un temps suffisant pour que pénètre la quantité requise de composé sus-mentionné.

L'invention concerne également l'application du procédé de séparation de la Lp(a) décrit ci-dessus, à la détermination, chez l'Homme, du risque athérogène lié à la présence de la Lp(a) dans l'organisme.

A ce titre, l'invention a pour objet une méthode de détermination in vitro du risque athérogène lié à la présence de la Lp(a), cette méthode étant réalisée à partir d'un échantillon biologique prélevé chez l'homme, notamment à partir de sérum ou de plasma, et comprenant:

- le dépôt d'une quantité appropriée de l'échantillon biologique sous forme liquide sur un gel d'électrophorèse contenant au moins un composé susceptible de modifier de façon différentielle la mobilité électrophorétique de la Lp (a) par rapport aux autres lipoprotéines, notamment un gel tel que défini ci-dessus, ou sur un gel préparé extemporanément, à partir d'un gel classique ne comprenant pas de composé tel que défini ci-dessus, par immersion, pendant un temps suffisant, de ce gel classique dans une solution de ce composé pour que pénètre la quantité requise de composé sus-mentionné, notamment dans une solution contenant des cations ou complexes cationiques choisis notamment parmi ceux cités ci-dessus, ou des molécules ayant une partie hydrophobe et chargées négativement choisies notamment parmi celles citées ci-dessus ou des agents tensio-actifs neutres choisis notamment parmi ceux cités ci-dessus,
- une étape de migration électrophorétique,
- la détection de la Lp(a) éventuellement présente dans l'échantillon biologique, notamment à l'aide d'un colorant spécifique des lipoprotéines tel que le colorant Noir Soudan, Rouge Soudan, ou de réactifs enzymatiques spécifiques des lipides ou d'anticorps dirigés contre les lipoprotéines, et en particulier contre la Lp(a).

L'invention concerne également une méthode de détermination in vitro du risque athérogène lié à la présence de la Lp(a), cette méthode étant réalisée à partir d'un échantillon biologique prélevé chez l'homme, notamment à partir de sérum ou de plasma, et comprenant:

- l'incubation de l'échantillon biologique prélevé avec une solution contenant des molécules ayant une partie hydrophobe et chargées négativement choisies notamment parmi celles citées ci-dessus ou des agents tensio-actifs neutres, notamment ceux cités ci-dessus,
- le dépôt d'une quantité appropriée de l'échantillon biologique ainsi traité sur un gel classique pour électrophorèse,
- une étape de migration électrophorétique,
- la détection de la Lp(a) éventuellement présente dans l'échantillon biologique, notamment à l'aide d'un colorant spécifique des lipoprotéines tel que le colorant Noir Soudan, Rouge Soudan, ou de réactifs enzymatiques spécifiques des lipides, ou d'anticorps dirigés contre les lipoprotéines, et en particulier contre la Lp(a).

La position de la Lp(a), qui peut être repérée par un anticorps spécifique anti-Lp(a), est, en pratique courante mettant en oeuvre le procédé de l'invention, révélée par un colorant spécifique des lipoprotéines tel que le Noir Soudan, le Rouge Soudan ou des réactifs enzymatiques spécifiques des lipides.

Ces méthodes de détermination du risque athérogène comprennent avantageusement une étape supplémentaire de dosage de la Lp(a), notamment selon la méthode de LAURELL et al publiée dans Anal. Biochem. (1966) 15:45-52. Le seuil de sensibilité de la méthode de diagnostic de l'invention est de l'ordre de 0,15 g/l de Lp(a) plasmatique, le taux pathologique de Lp(a) étant de l'ordre de 0,3 g/l.

Le procédé selon l'invention permet de façon intéressante, la séparation de la Lp(a) sous sa forme native, des différentes lipoprotéines présentes sous leur forme native dans le milieu biologique déposé sur le gel, et ce par une électrophorèse suivie d'une révélation colorée, sans faire nécessairement appel à la technique d'immunoblot.

Lors de la réalisation du procédé selon l'invention, la Lp(a) est située entre les fractions VLDL et HDL, se trouve ainsi dans une zone parfaitement résolue sur le gel et dépourvue de traînées (donc dépourvue de bruit de fond).

De plus selon le procédé de l'invention, la fraction IDL parfois présente dans la l'hyperlipidémie de type III est située entre la fraction LDL et VLDL et ne peut en aucun cas être confondue avec la Lp(a).

Enfin dans le cas d'échantillons de sérum vieillis, le ralentissement de la fraction VLDL (préß) ne peut en aucun cas masquer la Lp(a) qui précède la fraction VLDL.

Par la technique d'électrophorèse sur gel classique et comme conséquence des différents inconvénients décrits plus haut il est mentionné que la lecture densitométrique du lipidogramme présente une moindre spécificité et sensibilité que l'examen visuel de l'analyse.

La corrélation entre les valeurs de Lp(a) obtenues par densitométrie sur gel classique avec une technique de dosage de la Lp(a) par immunonéphélémétrie est seulement de $\pi = 0,55$ (Clinica Chimica Acta, 188 -1990-p71-78).

Selon le procédé que nous proposons, la corrélation entre la densitométrie du lipidogramme et une technique de dosage de la Lp(a) par électroimmunodiffusion selon Laurell montre un coefficient de corrélation $\pi = 0,91$ (sur 30 sérums Lp(a) positifs).

Des kits appropriés pour la mise en oeuvre d'un procédé ou d'une méthode de diagnostic selon l'invention, comprennent:

- un gel pour électrophorèse, notamment un gel d'agarose,
- une, ou plusieurs, solution(s) de composés, ces composés étant notamment des cations ou complexes cationiques choisis notamment parmi ceux cités ci-dessus, ou des molécules hydrophobes chargées négativement choisies notamment parmi celles citées ci-dessus ou des agents tensio-actifs neutres choisis notamment parmi ceux cités ci-dessus,
- la ou les solutions de tampons nécessaires à la migration électrophorétique des lipoprotéines sur le gel,
- les réactifs permettant de détecter les lipoprotéines, dont la Lp(a), après migration sur le gel, notamment le colorant Noir Soudan, Rouge Soudan, ou de réactifs enzymatiques spécifiques des lipides.

La solution utilisée dans le kit

- soit sert à retamponner le gel,
- soit est ajoutée dans l'échantillon biologique.

De tels kits comprennent :

- un gel selon l'invention comprenant au moins un composé susceptible de modifier de façon différentielle la mobilité électrophorétique de la Lp(a) par rapport aux autres lipoprotéines, notamment un gel tel que défini ci-dessus,
- la ou les solutions de tampons nécessaires à la migration électrophorétique des lipoprotéines sur le gel,
- les réactifs permettant de détecter les lipoprotéines, dont la Lp(a), après migration sur le gel, notamment le colorant Noir Soudan, Rouge Soudan, ou de réactifs enzymatiques spécifiques des lipides.

Légende des figures :

- Figures 1A et 1B :

Les figures 1A et 1B représentent un lipidogramme obtenu sur un gel témoin (ne contenant pas de composé susceptible de modifier de façon différentielle la mobilité électrophorétique de la Lp(a), par rapport à celle des autres lipoprotéines) d'agarose 0,5 %, le tampon de migration utilisé étant le suivant :

- Tris 0,06 M, Véronal 0,01 M, Véronal sodé 0,05 M, BSA 1 g/l.

Sur les 2 pistes de gauche (pistes 1 et 2), les sérums déposés possèdent un taux de Lp(a) pathologique. Sur les 2 pistes de droite (pistes 3 et 4), les sérums déposés ont un taux de Lp(a) nul ou très faible.

La Figure 1A représente la révélation des lipoprotéines avec du Noir Soudan.

La Figure 1B représente l'immunofixation avec un antisérum spécifique dirigé contre l'Apo(a) et permet de mettre en évidence la Lp(a) sur les pistes 1 et 2 qui n'était pas visible sur le lipidogramme de la Figure 1A, car la Lp(a) est superposée à la fraction VLDL.

- Figures 2A et 2B :

Les figures 2A et 2B représentent un lipidogramme obtenu sur un gel de l'invention contenant 0,03 M d'acétate de magnésium (le tampon est identique à celui décrit dans la Figure 1A). Sur ces gels sont disposés les mêmes sérums que sur les Figures 1A et 1B et aux mêmes positions.

La Figure 2A représente la révélation des lipoprotéines avec du Noir Soudan.

Sur les deux pistes 1 et 2 de la Figure 2A, on observe la présence d'une bande qui ne figurait pas sur la Figure 1A et qui correspond à la Lp(a).

La Figure 2B représente l'immunofixation avec un antisérum spécifique dirigé contre l'Apo(a) et confirme la révélation de la Lp(a), qui apparaît également dans la Figure 2A.

- Figure 3 :

La Figure 3 représente un lipidogramme obtenu avec un gel identique à celui des figures 2A et 2B, dans des conditions de migration identiques à celles utilisées dans le cadre des figures 2A et 2B.

Un sérum possédant un taux pathologique en Lp(a), a été déposé sur les 6 pistes.

Sur les pistes 1 à 5, on a effectué des immunofixations, avec les antisérums suivants:

piste 1 : anti-apoE
piste 2 : anti-apoCIII
piste 3 : anti-apo AI
piste 4 : anti-apo(a)
piste 5 : anti-apoB
piste 6 : on a effectué la coloration des lipoprotéines au Noir Soudan.

On constate que la Lp(a) (constituée d'apo(a) et d'apoB) est séparée des VLDL et LDL, et n'est pas dégradée.

- Figure 4 :

La Figure 4 représente un gel contenant de l'acide hydroxy-1 naphtalène carboxylique-2 à 0,026 M. Huit sérums différents contenant des taux allant de 0 à 0,7 g/l de Lp(a) ont été déposés.

La migration a lieu à 50 V pendant 45 mn. La révélation des lipoprotéines est effectuée à l'aide de Noir Soudan.

On constate la présence de la Lp(a) située en arrière de la fraction LDL, et de façon bien différenciée.

- Figures 5A et 5B :

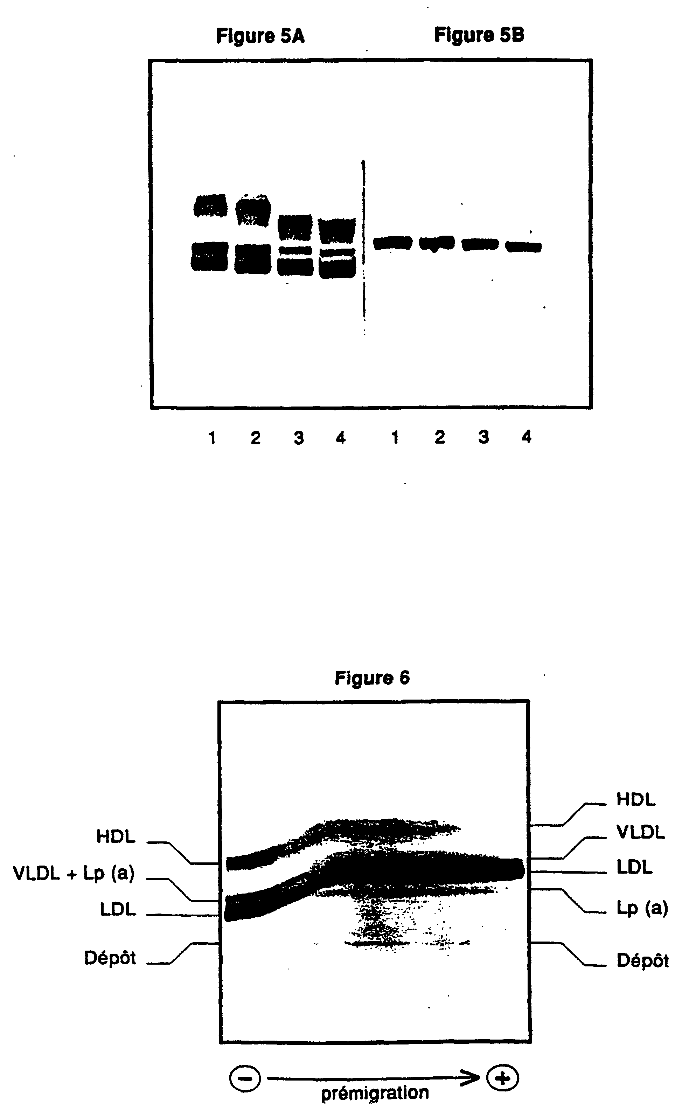

Les Figures 5A et 5B représentent un gel tel que défini pour la Figure 1A, sur lequel on a déposé les quatre échantillons suivants :

. piste 1 : sérum non traité,
. piste 2 : sérum traité au Triton X-100 $10^{-3}$ M,
. piste 3 : sérum traité au Tween 20 $10^{-3}$ M,
. piste 4 : sérum traité au BRIJ 35 $10^{-3}$ M.

L'électrophorèse a été effectuée dans les conditions décrites à propos de la Figure 1A.

Sur la Figure 5A, les lipoprotéines ont été colorées au Noir Soudan. Sur la Figure 5B, on a effectué une immunofixation avec un antisérum spécifique dirigé contre la Lp(a).

On constate que les détergents neutres ont un effet de freinage sur les lipoprotéines, la Lp(a) étant beaucoup moins affectée que les autres fractions lipoprotéiques.

- Figure 6 :

La Figure 6 représente l'électrophorèse d'un sérum contenant de la Lp(a), sur un gel dans lequel la partie droite comprend une molécule ayant une partie hydrophobe et chargée négativement (acide hydroxy-1 naphtalène carboxylique-2) et dans lequel la partie gauche ne comprend pas ce composé et correspond donc à une partie de gel témoin.

Cette expérience montre directement l'effet de l'acide hydroxy-1 naphtalène carboxylique-2 sur les différentes fractions lipoprotéiques : les HDL, VLDL et LDL sont fortement accélérées, alors que la Lp(a) garde sensiblement la même mobilité.

## EXEMPLE I : DESCRIPTION DES MANIPULATIONS CORRESPONDANT AUX FIGURES 1 ET 2

1) Préparation des gels des figures 1A et 1B :

La composition du gel est la suivante :

- agarose 0,5 %, Tris 0,06 M, Véronal 0,01 M, Véronal sodé 0,05 M, albumine bovine 0,1%.

Dans un Erlenmeyer de 100 ml, on introduit 39 ml d'eau déminéralisée et on ajoute sous agitation magnétique 0,25 g d'agarose. On porte à ébullition pendant 5 mn sur plaque chauffante et sous agitation constante. L'agarose est alors dissout, donnant une solution parfaitement limpide. On équilibre ensuite la température de la solution à 50°C dans un bain thermostaté.

Dans un Erlenmeyer de 50 ml, on introduit 10 ml d'eau déminéralisée et on ajoute sous agitation magnétique 0,36 g de Tris, 0,092 g de Véronal, 0,515 g de Véronal sodé. Après dissolution complète, l'ensemble est stabilisé à 50°C dans le bain thermostaté.

Dans un tube à hémolyse, on introduit 1 ml d'eau déminéralisée et 0,05 g d'albumine bovine. La dissolution est accélérée à l'aide d'un vortex. La solution est portée à 50°C.

Dans l'Erlenmeyer de 100 ml contenant la solution d'agarose, on ajoute sous agitation magnétique les solutions concentrées de tampon et d'albumine bovine. L'ensemble est bien homogénéisé et maintenu à 50°C.

Le gel est ensuite coulé. Pour ce faire, 5 ml de cette solution sont répartis uniformément sur un film plastique hydrophile de dimensions 10 x 8 cm.

2) Préparation des gels des figures 2A ET 2B :

La composition du gel est la suivante :

- agarose 0,5%, Tris 0,06 M, Véronal 0,01 M, Véronal sodé 0,05 M, acétate de magnésium 0,03 M, albumine bovine 0,1%.

La préparation du gel suit le même protocole que celui des figures 1A et 1B, à la différence près que l'on ajoute 0,322 g d'acétate de magnésium tétrahydraté à la solution tampon concentré.

3) Conditions de migration :

Les sérums frais sont déposés à 2,5 cm du bord cathodique du gel (dépôts de 3 μl).

La migration est réalisée en tampon Tris 0,06 M, Véronal 0,01 M, Véronal sodé 0,05 M à 50 V pendant 45 mn.

Dans le cas des figures 1A et 1B, la migration dure pendant 45 mn.

Dans le cas des figures 2A et 2B, la migration est poursuivie pendant 1 h.

Après la migration, le gel est séché complètement et coloré avec un colorant des lipides (Noir Soudan) (figures 1A et 2A), ou soumis à une immunofixation avec un antisérum spécifique dirigé contre la Lp(a) (figures 1B et 2B).

## EXEMPLE II : DESCRIPTION DES MANIPULATIONS CORRESPONDANT A LA FIGURE 6 :

Le gel est identique à celui de la Figure 4. Une prémigration d'une heure à 50 V est effectuée dans le sens indiqué sur la Figure. Au cours de cette prémigration, l'anion se déplace vers l'anode, de sorte que la partie cathodique du gel s'en trouve dépourvue.

Un dépôt unique d'un sérum contenant de la Lp(a) est ensuite pratiqué sur toute la longueur du gel, et la migration s'effectue dans le sens perpendiculaire à celui de la prémigration pendant 45 mn. Les lipoprotéines sont ensuite colorées au Noir Soudan.

## EXEMPLE III : COMPARAISON DES DISTANCES DE MIGRATION DES FRACTIONS LIPOPROTEIQUES SUR DIFFERENTS GELS : d distance de migration sur le gel contenant le composé $d_T$ distance de migration sur le gel témoin

1) Gel de l'invention avec acétate de magnésium 0,03 M (gel de la Figure 2):

| Fraction lipoprotéique | HDL | VLDL | LDL | Lp(a) |
|---|---|---|---|---|
| $\frac{d}{d_T}$ | 0,75 | 0,61 | 0,5 | 0,96 |

2) Gel de l'invention contenant l'acide hydroxy-1 naphtalène carboxylique-2 0,026 M (gel de la Figure 4):

| Fraction lipoprotéique | HDL | VLDL | LDL | Lp(a) |
|---|---|---|---|---|
| $\frac{d}{d_T}$ | 1,42 | 1,9 | 2,67 | 1,1 |

EXEMPLE IV

Un gel classique de même composition que celui obtenu dans l'exemple I correspondant aux figures 1A et 1B est soumis avant le dépôt des échantillons à une préélectrophorèse avec le tampon Tris 0,06M, Veronal acide 0,01 M Veronal sodé 0,05 M, albumine bovine 0,1% auquel a été ajouté dans le bac cathodique, l'acide hydroxy 1 naphtalènicarboxylique 2 à la concentration de 0,026M.

Après 30' de migration sous 50 Volts correspondant à un gradient de potentiel d'environ 6 V/cm, la pénétration dans le gel de l'acide hydroxy 1 naphtalène carboxylique 2 est de 40 mm à partir du bac cathodique. Les échantillons sont alors déposés dans cette zone par exemple à 30 mm du bord cathodique du gel et la migration des échantillons est alors réalisée avec les mêmes tampons pendant 45' sous une tension de 50 V.

Les lipidogrammes obtenus sont identiques à ceux obtenus sur le gel de la figure 4.

EXEMPLE V

Un gel classique de même composition que celui de l'exemple I figures 1A et 1B est soumis avant le dépôt des échantillons à une préélectrophorèse avec le tampon de migration Tris 0,6M, Veronal acide 0,01M, veronal sodé 0,05 M, albumine bovine 0,1% auquel a été ajouté dans le bac anodique $5 \cdot 10^{-2}$ M d'acétate de magnésium.

Après 45' de migration sous 50 V correspondant à un gradient de potentiel d'environ 6V/cm la pénétration dans le gel des ions $Mg^{2+}$ à partir du bac anodique est de 40 mm.

Les échantillons sont alors déposés dans une zone du gel qui n'a pas encore été atteinte par le front des ions $Mg^{2+}$ venant du bac anodique et située par exemple à 45 mm du bord anodique du gel. La migration est alors réalisée avec les mêmes tampons pendant 1 h sous une tension de 50 V. Le profil obtenu est analogue à celui obtenu sur les gels des figures 2A et 2B avec en particulier la fraction lpa précédant la fraction VLDL mais avec une meilleure séparation entre la fraction LDL et le point de dépôt des échantillons.

**Revendications**

1. Procédé de séparation par électrophorèse de la Lp(a) et des différentes lipoprotéines contenues dans un milieu biologique, **caractérisé en ce que** la migration électrophorétique des lipoprotéines est réalisée dans des conditions telles que le gel d'électrophorèse et/ou le milieu biologique susmentionné contient des composés susceptibles de modifier de façon différentielle la mobilité electrophorétique de la Lp(a), par rapport à celle des autres lipoprotéines, lesdits composés étant choisis parmi les molécules ayant une partie hydrophobe et chargées négativement, les agents tensio-actifs neutres, les cations ayant un degré d'oxydation supérieur ou égal à 2 et les complexes cationiques $NH_4^+$, $(NH_4^+)_2Fe^{2+}$, $NH_4^+Fe^{3+}$.

2. Procédé selon la revendication 1, **caractérisé en ce que** le susdit composé permet de séparer, dans un milieu biologique, la fraction Lp(a) des fractions VLDL et LDL.

3. Procédé selon la revendication 1 ou 2, **caractérisé en ce que** ledit composé permet de séparer, dans un milieu biologique, la fraction Lp(a) des fractions VLDL et LDL, tout en conservant la séparation des HDL, LDL et VLDL.

4. Procédé selon l'une quelconque des revendications 1 à 3, **caractérisé en ce que** les cations ou complexes cationiques sont ceux dont les hydroxydes, à pH allant d'environ 8 à environ 9, ne sont pas précipités ou sont partiellement précipités, de telle sorte qu'il y ait au moins $10^{-3}$ moles/l de cations ou de complexes cationiques non précipités dans le gel pour électrophorèse.

EP 0 546 916 B1

**5.** Procédé selon l'une quelconque des revendications 1 à 4, **caractérisé en ce que** les cations sont choisis parmi les colonnes IIa, IIIa et IIIb du tableau de Mandeleiv.

**6.** Procédé selon l'une quelconque des revendications 1 à 4, **caractérisé en ce que** les cations sont choisis parmi $Mg^{2+}$, $Ca^{2+}$, $Ba^{2+}$, $Sr^{2+}$, $Mn^{2+}$, $Be^{2+}$, $Al^{3+}$, $La^{3+}$, $Ce^{3+}$, $In^{3+}$ et $Y^{3+}$.

**7.** Procédé selon l'une quelconque des revendications 1 à 6, **caractérisé en ce que** le cation est $Mg^{++}$.

**8.** Procédé selon l'une quelconque des revendications 1 à 7, **caractérisé en ce que** la concentration des cations ou complexes cationiques, dans le gel d'électrophorèse, est de l'ordre de $10^{-3}$ M à $10^{-1}$ M.

**9.** Procédé selon l'une quelconque des revendications 1 à 8, **caractérisé en ce que** la concentration des cations ou complexes cationiques, dans le gel d'électrophorèse est de l'ordre de $5 \times 10^{-3}$ M à $3 \times 10^{-2}$ M.

**10.** Procédé selon l'une quelconque des revendications 1 à 3, **caractérisé en ce que** le composé susceptible de modifier de façon différentielle la mobilité électrophorétique de la Lp(a) par rapport aux autres lipoprotéines est une molécule ayant une partie hydrophobe et chargée négativement.

**11.** Procédé selon la revendication 10, **caractérisé en ce que** la molécule ayant une partie hydrophobe et chargée négativement,

- • soit fait partie de la classe des agents tensio-actifs anioniques et comprend

  - \* soit une chaîne aliphatique linéaire ou ramifiée de 3 à 10 atomes de carbone, comportant éventuellement des hétéroatomes, comportant une fonction acide telle que sulfurique, sulfonique, carboxylique ou phosphorique, et possédant une chaîne aliphatique condensée comportant au moins 1 à 6 cycles saturés de 5 ou 6 atomes de carbone,
  - \* soit une chaîne aliphatique linéaire ou ramifiée d'au moins 8 atomes de carbone, cette chaîne étant le cas échéant substituée par un (ou plusieurs) cycle(s) ou hétérocycle(s) aromatique(s), ladite chaîne ou ce (ou ces) cycle(s) ou hétérocycle(s) aromatique(s) étant substitué(s) par une fonction acide telle que carboxylique, sulfonique, sulfurique ou phosphorique,

- • soit est un dérivé aromatique chargé négativement et comportant au moins deux cycles aromatiques condensés, ou un cycle aromatique condensé à un hétérocycle aromatique, ou deux hétérocycles aromatiques condensés, l'un au moins des cycles comportant une fonction acide telle que carboxylique, sulfonique, sulfurique ou phosphorique et l'autre cycle ne comportant pas de fonction susceptibles de faire perdre à l'ensemble de la molécule son caractère hydrophobe.

**12.** Procédé selon la revendication 10 ou 11, **caractérisé en ce que** la molécule ayant une partie hydrophobe et chargée négativement fait partie de la classe des agents tensio-actifs anioniques et comprend une chaîne aliphatique linéaire ou ramifiée de 10 à 18 atomes de carbone.

**13.** Procédé selon la revendication 11, **caractérisé en ce que** le dérivé aromatique comportant deux cycles aromatiques est le naphtalène ou le cycle aromatique condensé à un hétérocycle aromatique est la quinoléine.

**14.** Procédé selon la revendication 11, **caractérisé en ce que** l'autre cycle ne comporte pas de charge polaire.

**15.** Procédé selon l'une quelconque des revendications 10 à 14 **caractérisé en ce que** la molécule ayant une partie hydrophobe et chargée négativement est choisie parmi les composés suivants :

- - sodium dodécylsulfate,
- - sodium dodécylbenzènesulfonate,
- - cholate de sodium,
- - acide naphtalène carboxylique-2,
- - acide naphtalène sulfonique-2,
- - acide amino-2 naphtalène sulfonique-1,
- - acide hydroxy-4 naphtalène sulfonique-1,
- - acide amino-1 naphtalène sulfonique-5,

- Noir Amido,
- Violet Acide 17,
- Bleu de Coomassie R250,
- Palatine fast black wan,
- acide hydroxy-1 naphtalène carboxylique-2, et
- l'acide hydroxy-3 naphtalène carboxylique-2.

16. Procédé selon la revendication 10 ou 11, **caractérisé en ce que** la molécule ayant une partie hydrophobe et chargée négativement est l'acide hydroxy-1 naphtalène carboxylique-2 ou l'acide hydroxy-3 naphtalène carboxylique-2.

17. Procédé selon l'une quelconque des revendications 1 à 3, **caractérisé en ce que** le composé susceptible de modifier de façon différentielle la mobilité électrophorique de la Lp(a) par rapport aux autres lipoprotéines est un agent tensio-actif neutre (ou détergent neutre).

18. Procédé selon la revendication 17, dans lequel l'agent tensio-actif neutre est tel qu'il est soluble en phase aqueuse.

19. Procédé selon la revendication 17 ou 18, **caractérisé en ce que** l'agent tensio-actif neutre est choisi parmi le Triton X100®, Triton X405®, le Tween 20®, le NP40® et le BRIJ35®.

20. Procédé selon l'une quelconque des revendications 11 à 19, **caractérisé en ce que** la concentration des agents tensio-actifs anioniques ou des agents tensio-actifs neutres dans le gel d'électrophorèse, est de l'ordre de $10^{-6}$ à $10^{-3}$ M et s'il sont introduits préalablement dans le milieu biologique de l'ordre de $10^{-4}$ à $10^{-1}$ M.

21. Procédé selon la revendication 20, **caractérisé en ce que** la concentration des agents tensio-actifs anioniques ou neutres dans le gel d'électrophorèse est de $10^{-4}$ à $10^{-3}$ M.

22. Procédé selon la revendication 20, **caractérisé en ce que** les tensio-actifs anioniques ou neutres sont introduits préalablement dans le milieu biologique et la concentration est de l'ordre de $10^{-3}$ à $10^{-2}$ M.

23. Procédé selon l'une des quelconques revendications 11 à 16, **caractérisé en ce que** la concentration des dérivés aromatiques chargés négativement dans le gel est d'environ $10^{-3}$ à $10^{-1}$ M, et s'ils sont introduits préalablement dans le milieu biologique, d'environ $10^{-2}$ M à environ 1 M.

24. Procédé selon la revendication 23, **caractérisé en ce que** la concentration des dérivés aromatiques chargés négativement dans le gel est d'environ $10^{-2}$ à $5.10^{-2}$ M.

25. Procédé selon la revendication 23, **caractérisé en ce que** la concentration des dérivés aromatiques chargés négativement sont introduits préalablement dans le milieu biologique et la concentration est d'environ $5.10^{-2}$ à environ $5.10^{-1}$ M.

26. Application du procédé selon l'une quelconque des revendications 1 à 25, à la détermination, chez l'homme, du risque athérogène lié à la présence de la Lp(a) dans l'organisme.

27. Méthode de détermination in vitro du risque athérogène lié à la présence de la Lp(a), cette méthode étant réalisée à partir d'un échantillon biologique prélevé chez l'homme, et comprenant :

- le dépôt d'une quantité appropriée de l'échantillon biologique sous forme liquide sur un gel d'électrophorèse,
- une séparation par électrophorèse de la Lp(a) et des différentes lipoprotéines contenues dans l'échantillon biologique, selon l'une quelconque des revendications 1 à 25,
- la détection de la Lp(a) éventuellement présente dans l'échantillon biologique.

28. Méthode de détermination in vitro du risque athérogène lié à la présence de la Lp(a), selon la revendication 27, cette méthode étant réalisée à partir d'un échantillon biologique prélevé chez l'homme, dans laquelle le gel d'électrophorèse contient au moins un composé susceptible de modifier de façon différentielle la mobilité électrophorétique de la Lp(a) par rapport aux autres lipoprotéines, tel que défini dans l'une quelconque des revendications 1 à 25.

**29.** Méthode de détermination in vitro du risque athérogène lié à la présence de la Lp(a), selon la revendication 27, cette méthode étant réalisée à partir d'un échantillon biologique prélevé chez l'homme, et comprenant :

- l'incubation de l'échantillon biologique prélevé avec une solution contenant des molécules ayant une partie hydrophobe et chargées négativement ou des agents tensio-actifs neutres,
- le dépôt d'une quantité appropriée de l'échantillon biologique ainsi traité sur un gel classique pour électrophorèse,
- une étape de migration électrophorétique,
- la détection de la Lp(a) éventuellement présente dans l'échantillon biologique.

**30.** Méthode selon la revendication 29, **caractérisée en ce que** les molécules ayant une partie hydrophobe et chargées négativement sont définies à l'une quelconque des revendications 11 à 16.

**31.** Méthode selon la revendication 29, **caractérisé en ce que** les agents tensio-actifs neutres sont définis à la revendication 18 ou 19.

**32.** Méthode selon l'une quelconque des revendications 27 à 31, **caractérisée en ce que** l'échantillon biologique prélevé chez l'homme est du sérum ou du plasma.

**33.** Méthode de détermination du risque athérogène lié à la présence de la Lp(a) selon l'une quelconque des revendications 27 à 32 **caractérisée en ce que** la détection est réalisée à l'aide d'un colorant spécifique des lipoprotéines ou de réactifs enzymatiques spécifiques des lipides, ou d'anticorps marqués dirigés contre les lipoprotéines.

**34.** Méthode selon l'une quelconque des revendications 27 à 33, **caractérisée en ce que** la détection est réalisée à l'aide de Noir Soudan, Rouge Soudan, ou d'anticorps dirigés contre la Lp(a).

**35.** Méthode de détermination du risque athérogène lié à la présence de la Lp(a) selon l'une quelconque des revendications 27 à 34, **caractérisée en ce qu'**elle comprend une étape supplémentaire de dosage de la Lp(a).

**36.** Méthode de détermination du risque athérogène lié à la présence de la Lp(a) selon la revendication 35, **caractérisée en ce que** le dosage de la Lp(a) est réalisé selon la méthode de LAURELL et al.

**37.** Utilisation pour la séparation par électrophorèse de la Lp(a) et des différentes lipoprotéines contenues dans un milieu biologique des composants suivants :

- un gel d'électrophorèse contenant au moins un composé susceptible de modifier de façon différentielle la mobilité électrophorétique de la Lp(a) par rapport aux autres lipoprotéines tel que défini dans l'une quelconque des revendications 1 à 25, et
- la ou les solutions de tampons nécessaires à la migration électrophorétique des lipoprotéines sur le gel, et
- les réactifs permettant de détecter les lipoprotéines, dont la Lp(a), après migration sur le gel.

**38.** Utilisation pour la séparation par électrophorèse de la Lp(a) selon la revendication 37, **caractérisée en ce que** les réactifs permettant de détecter les lipoprotéines sont choisis parmi le colorant Noir Soudan, Rouge Soudan ou des réactifs enzymatiques spécifiques des lipides.

**39.** Utilisation selon la revendication 37 comprenant en outre :

- une, ou plusieurs, solution(s) desdits composés, la solution étant utilisée

  • soit pour retamponner le gel,
  • soit pour être ajoutée dans l'échantillon biologique.

**40.** Utilisation selon la revendication 39, **caractérisée en ce que** lesdits composés sont choisis parmi ceux des revendications 1, 11 ou 18.

**41.** Utilisation pour la séparation par électrophorèse de la Lp(a) et des différentes lipoprotéines selon la revendication 39 ou 40, **caractérisée en ce que** les réactifs permettant de détecter les lipoprotéines sont choisis parmi le colorant Noir Soudan, Rouge Soudan ou des réactifs enzymatiques spécifiques des lipides.

**Patentansprüche**

1. Verfahren zur Trennung durch Elektrophorese von Lp(a) und anderen Lipoproteinen, die in einem biologischen Medium enthalten sind, **dadurch gekennzeichnet, dass** die elektrophoretische Wanderung der Lipoproteine unter solchen Bedingungen durchgeführt wird, dass das Elektrophoresegel und/oder das oben erwähnte biologische Medium Verbindungen enthält, die fähig sind, die elektrophoretische Mobilität des Lp(a) im Verhältnis zu der der anderen Lipoproteine in differentieller Weise zu modifizieren, wobei die genannten Verbindungen ausgewählt werden aus den Molekülen, die einen hydrophoben Anteil aufweisen und negativ geladen sind, den neutralen Tensiden, den Kationen, die einen Oxidationsgrad größer oder gleich 2 aufweisen, und den kationischen Komplexen $NH_4^+$, $(NH_4^+)_2Fe^{2+}$, $NH_4^+Fe^{3+}$.

2. Verfahren nach Anspruch 1, **dadurch gekennzeichnet, dass** die oben genannte Verbindung es ermöglicht, in einem biologischen Medium die Fraktion Lp(a) von den Fraktionen VLDL und LDL zu trennen.

3. Verfahren nach Anspruch 1 oder 2, **dadurch gekennzeichnet, dass** die genannte Verbindung es ermöglicht, in einem biologischen Medium die Fraktion Lp(a) von den Fraktionen VLDL und LDL zu trennen, wobei die Trennung von HDL, LDL und VLDL beibehalten wird.

4. Verfahren nach irgendeinem der Ansprüche 1 bis 3, **dadurch gekennzeichnet, dass** die Kationen oder kationischen Komplexe diejenigen sind, deren Hydroxide bei einem pH, der von ca. 8 bis ca. 9 reicht, nicht ausgefällt werden oder teilweise ausgefällt werden, derart, dass es wenigstens $10^{-3}$ Mol/l Kationen oder kationische Komplexe gibt, die in dem Gel zur Elektrophorese nicht ausgefällt werden.

5. Verfahren nach irgendeinem der Ansprüche 1 bis 4, **dadurch gekennzeichnet, dass** die Kationen ausgewählt werden aus den Gruppen IIa, IIIa und IIIb des Periodensystems von Mendelejew.

6. Verfahren nach irgendeinem der Ansprüche 1 bis 4, **dadurch gekennzeichnet, dass** die Kationen ausgewählt werden aus $Mg^{2+}$, $Ca^{2+}$, $Ba^{2+}$, $Sr^{2+}$, $Mn^{2+}$, $Be^{2+}$, $Al^{3+}$, $La^{3+}$, $Ce^{3+}$, $In^{3+}$ und $Y^{3+}$.

7. Verfahren nach irgendeinem der Ansprüche 1 bis 6, **dadurch gekennzeichnet, dass** das Kation $Mg^{++}$ ist.

8. Verfahren nach irgendeinem der Ansprüche 1 bis 7, **dadurch gekennzeichnet, dass** die Konzentration der Kationen oder kationischen Komplexe in dem Elektrophoresegel in der Größenordnung von $10^{-3}$ M bis $10^{-1}$ M liegt.

9. Verfahren nach irgendeinem der Ansprüche 1 bis 8, **dadurch gekennzeichnet, dass** die Konzentration der Kationen oder kationischen Komplexe in dem Elektrophoresegel in der Größenordnung von $5 \times 10^{-3}$ M bis $3 \times 10^{-2}$ M liegt.

10. Verfahren nach irgendeinem der Ansprüche 1 bis 3, **dadurch gekennzeichnet, dass** die Verbindung, die fähig ist, die elektrophoretische Mobilität des Lp(a) im Verhältnis zu den anderen Lipoproteinen in einer differentiellen Weise zu modifizieren, ein Molekül ist, das einen hydrophoben Anteil aufweist und negativ geladen ist.

11. Verfahren nach Anspruch 10, **dadurch gekennzeichnet, dass** das Molekül, das einen hydrophoben Anteil aufweist und negativ geladen ist,

- entweder aus der Klasse der anionischen Tenside stammt und umfasst

  * entweder eine lineare oder verzweigte aliphatische Kette mit 3 bis 10 Kohlenstoffatomen, welche gegebenenfalls Heteroatome aufweist, welche eine Säurefunktion wie beispielsweise der von einer Schwefelsäure, Sulfonsäure, Carbonsäure oder Phosphorsäure aufweist, und welche eine kondensierte aliphatische Kette besitzt, die wenigstens 1 bis 6 gesättigte Zyklen mit 5 oder 6 Kohlenstoffatomen umfasst,

  * oder eine lineare oder verzweigte aliphatische Kette mit wenigstens 8 Kohlenstoffatomen, wobei diese Kette gegebenenfalls durch einen (oder mehrere) aromatischen Zyklus (Zyklen) oder Heterozyklus(en) substituiert ist, wobei die genannte Kette oder der (oder die) aromatische(n) Zyklus (Zyklen) oder Heterozyklus(en) durch eine Säurefunktion wie beispielsweise der von einer Carbonsäure, Sulfonsäure, Schwefelsäure oder Phosphorsäure substituiert ist,

- oder ein aromatisches Derivat ist, das negativ geladen ist und wenigstens zwei kondensierte aromatische Zyklen oder einen aromatischen Zyklus, der mit einem aromatischen Heterozyklus kondensiert ist, oder zwei aromatische kondensierte Heterozyklen aufweist, wobei wenigstens einer der Zyklen eine Säurefunktion wie beispielsweise die von einer Carbonsäure, Sulfonsäure, Schwefelsäure oder Phosphorsäure aufweist und der andere Zyklus keine Funktion aufweist, die dazu geeignet ist, dass die Gesamtheit des Moleküls ihren hydrophoben Charakter verliert.

12. Verfahren nach Anspruch 10 oder 11, **dadurch gekennzeichnet, dass** das Molekül, das einen hydrophoben Anteil aufweist und negativ geladen ist, aus der Klasse der anionischen Tenside stammt und eine lineare oder verzweigte aliphatische Kette mit 10 bis 18 Kohlenstoffatomen umfasst.

13. Verfahren nach Anspruch 11, **dadurch gekennzeichnet, dass** das aromatische Derivat, welches zwei aromatische Zyklen aufweist, Naphthalin ist, oder der aromatische Zyklus, der mit einem aromatischen Heterozyklus kondensiert ist, Chinolin ist.

14. Verfahren nach Anspruch 11, **dadurch gekennzeichnet, dass** der andere Zyklus keine polare Ladung aufweist.

15. Verfahren nach irgendeinem der Ansprüche 10 bis 14, **dadurch gekennzeichnet, dass** das Molekül, das einen hydrophoben Anteil aufweist und negativ geladen ist, ausgewählt wird aus den folgenden Verbindungen:

   - Natriumdodecylsulfat,
   - Natriumdodecylbenzolsulfonat,
   - Natriumcholat,
   - 2-Naphthalincarbonsäure,
   - 2-Naphthalinsulfonsäure,
   - 2-Amino-1-naphthalinsulfonsäure,
   - 4-Hydroxy-1-napthalinsulfonsäure,
   - 1-Amino-5-naphthalinsulfonsäure,
   - Amidoschwarz,
   - Violet Acide 17,
   - Coomassie-Blau R250,
   - Palatine Fast Black Wan,
   - 1-Hydroxy-2-naphthalincarbonsäure und
   - 3-Hydroxy-2-naphthalincarbonsäure.

16. Verfahren nach Anspruch 10 oder 11, **dadurch gekennzeichnet, dass** das Molekül, welches einen hydrophoben Anteil aufweist und negativ geladen ist, 1-Hydroxy-2-naphthalincarbonsäure oder 3-Hydroxy-2-naphthalincarbonsäure ist.

17. Verfahren nach irgendeinem der Ansprüche 1 bis 3, **dadurch gekennzeichnet, dass** die Verbindung, die fähig ist, die elektrophoretische Mobilität des Lp(a) im Verhältnis zu den anderen Lipoproteinen in differentieller Weise zu modifizieren, ein neutrales Tensid (oder ein neutrales Detergens) ist.

18. Verfahren nach Anspruch 17, bei welchem das neutrale Tensid derart ist, dass es in einer wässrigen Phase löslich ist.

19. Verfahren nach Anspruch 17 oder 18, **dadurch gekennzeichnet, dass** das neutrale Tensid ausgewählt wird aus Triton X100®, Triton X405®, Tween 20®, NP40® und BRIJ35®.

20. Verfahren nach irgendeinem der Ansprüche 11 bis 19, **dadurch gekennzeichnet, dass** die Konzentration der anionischen Tenside oder der neutralen Tenside in dem Elektrophoresegel in der Größenordnung von $10^{-6}$ bis $10^{-3}$ M liegt und dass diese vorher in das biologische Medium in der Größenordnung von $10^{-4}$ bis $10^{-1}$ M eingeführt werden.

21. Verfahren nach Anspruch 20, **dadurch gekennzeichnet, dass** die Konzentration der anionischen oder neutralen Tenside in dem Elektrophoresegel $10^{-4}$ bis $10^{-3}$ M beträgt.

22. Verfahren nach Anspruch 20, **dadurch gekennzeichnet, dass** die anionischen oder neutralen Tenside vorher in das biologische Medium eingeführt werden und die Konzentration in der Größenordnung von $10^{-3}$ bis $10^{-2}$ M liegt.

23. Verfahren nach irgendeinem der Ansprüche 11 bis 16, **dadurch gekennzeichnet, dass** die Konzentration der negativ geladenen aromatischen Derivate in dem Gel ungefähr $10^{-3}$ bis $10^{-1}$ M beträgt und dass diese vorher bei ungefähr bei $10^{-2}$ M bis ungefähr 1 M in das biologische Medium eingeführt werden.

24. Verfahren nach Anspruch 23, **dadurch gekennzeichnet, dass** die Konzentration der negativ geladenen aromatischen Derivate in dem Gel ungefähr $10^{-2}$ bis $5 \times 10^{-2}$ M beträgt.

25. Verfahren nach Anspruch 23, **dadurch gekennzeichnet, dass** [die Konzentration] die negativ geladenen aromatischen Derivate vorher in das biologische Medium eingeführt werden und die Konzentration ungefähr $5 \times 10^{-2}$ bis ungefähr $5 \times 10^{-1}$ M beträgt.

26. Anwendung des Verfahrens nach irgendeinem der Ansprüche 1 bis 25 auf die Bestimmung des Atherogen-Risikos beim Menschen, das mit dem Vorliegen von Lp(a) im Organismus verbunden ist.

27. Verfahren zur Feststellung in vitro des Atherogen-Risikos, das mit dem Vorliegen von Lp(a) verbunden ist, wobei das Verfahren, ausgehend von einer biologischen Probe, die einem Menschen entnommen wurde, durchgeführt wird und umfasst:

- die Auftragung einer geeigneten Menge der biologischen Probe in flüssiger Form auf ein Elektrophoresegel,

- eine Trennung durch Elektrophorese des Lp(a) und der anderen Lipoproteine, die in der biologischen Probe enthalten sind, nach irgendeinem der Ansprüche 1 bis 25,

- den Nachweis des Lp(a), das gegebenenfalls in der biologischen Probe vorliegt.

28. Verfahren zur Feststellung in vitro des Atherogen-Risikos, das mit dem Vorliegen von Lp(a) verbunden ist, nach Anspruch 27, wobei das Verfahren ausgehend von einer biologischen Probe, die einem Menschen entnommen wurde, durchgeführt wird, bei welchem das Elektrophoresegel wenigstens eine Verbindung enthält, die fähig ist, die elektrophoretische Mobilität von Lp(a) im Verhältnis zu den anderen Lipoproteinen in differentieller Weise zu modifizieren, so wie es in irgendeinem der Ansprüche 1 bis 25 definiert ist.

29. Verfahren zur Feststellung in vitro des Atherogen-Risikos, das mit dem Vorliegen von Lp(a) verbunden ist, nach Anspruch 27, wobei das Verfahren ausgehend von einer biologischen Probe, die einem Menschen entnommen wurde, durchgeführt wird und umfasst:

- die Inkubation der entnommenen biologischen Probe mit einer Lösung, welche Moleküle enthält, die einen hydrophoben Anteil aufweisen und negativ geladen sind, oder neutralen Tensiden,

- die Auftragung einer geeigneten Menge der so behandelten biologischen Probe auf ein klassisches Elektrophoresegel,

- einen Schritt der elektrophoretischen Wanderung,

- den Nachweis von Lp(a), das gegebenenfalls in der biologischen Probe vorliegt.

30. Verfahren nach Anspruch 29, **dadurch gekennzeichnet, dass** die Moleküle, welche einen hydrophoben Anteil aufweisen und negativ geladen sind, wie in irgendeinem der Ansprüche 11 bis 16 definiert sind.

31. Verfahren nach Anspruch 29, **dadurch gekennzeichnet, dass** die neutralen Tenside wie in Anspruch 18 oder 19 definiert sind.

32. Verfahren nach irgendeinem der Ansprüche 27 bis 31, **dadurch gekennzeichnet, dass** die biologische Probe, die einem Menschen entnommen wurde, Serum oder Plasma ist.

33. Verfahren zur Feststellung des Atherogen-Risikos, das mit dem Vorliegen von Lp(a) verbunden ist, nach irgendeinem der Ansprüche 27 bis 32, **dadurch gekennzeichnet, dass** der Nachweis mit Hilfe eines spezifischen Anfärbungsmittels für Lipoproteine oder spezifischer enzymatischer Reagenzien für Lipide oder markierter Antikörper, die gegen die Lipoproteine gerichtet sind, durchgeführt wird.

34. Verfahren nach irgendeinem der Ansprüche 27 bis 33, **dadurch gekennzeichnet, dass** der Nachweis mit Hilfe von Sudanschwarz, Sudanrot oder Antikörpern, die gegen das Lp(a) gerichtet sind, durchgeführt wird.

35. Verfahren zur Feststellung des Atherogen-Risikos, das mit dem Vorliegen von Lp(a) verbunden ist, nach irgendeinem der Ansprüche 27 bis 34, **dadurch gekennzeichnet, dass** es einen zusätzlichen Schritt der Bestimmung von Lp(a) umfasst.

36. Verfahren zur Feststellung des Atherogen-Risikos, das mit dem Vorliegen von Lp(a) verbunden ist, nach Anspruch 35, **dadurch gekennzeichnet, dass** die Bestimmung von Lp(a) nach dem Verfahren von LAURELL et al. durchgeführt wird.

37. Verwendung zur Trennung durch Elektrophorese von Lp(a) und anderen Lipoproteinen, die in einem biologischen Medium enthalten sind, der folgenden Bestandteile:

   - einem Elektrophoresegel, das wenigstens eine Verbindung enthält, die fähig ist, die elektrophoretische Mobilität von Lp(a) im Verhältnis zu den anderen Lipoproteinen in differentieller Weise zu modifizieren wie in irgendeinem der Ansprüche 1 bis 25 definiert ist, und

   - einer oder mehrerer Pufferlösungen, die zur elektrophoretischen Wanderung der Lipoproteine auf dem Gel erforderlich ist/sind, und

   - der Reagenzien, die ermöglichen, die Lipoproteine und damit das Lp(a), nach der Wanderung auf dem Gel nachzuweisen.

38. Verwendung zur Trennung durch Elektrophorese von Lp(a) nach Anspruch 37, **dadurch gekennzeichnet, dass** die Reagenzien, welche das Nachweisen der Lipoproteine ermöglichen, ausgewählt werden aus den Farbstoffen Sudanschwarz, Sudanrot oder enzymatischen Reagenzien, die für Lipide spezifisch sind.

39. Verwendung nach Anspruch 37, die im übrigen umfasst:

   - eine oder mehrere Lösung(en) der genannten Verbindungen, wobei die Lösung verwendet wird

      * entweder, um das Gel wieder in einen Puffer zu überführen,

      * oder um der biologischen Probe zugefügt zu werden.

40. Verwendung nach Anspruch 39, **dadurch gekennzeichnet, dass** die genannten Verbindungen ausgewählt werden aus denen der Ansprüche 1, 11 oder 18.

41. Verwendung zur Trennung durch Elektrophorese von Lp(a) und anderen Lipoproteinen nach Anspruch 39 oder 40, **dadurch gekennzeichnet, dass** die Reagenzien, welche den Nachweis der Lipoproteine ermöglichen, ausgewählt werden aus den Farbstoffen Sudanschwarz, Sudanrot oder enzymatischen Reagenzien, die für Lipide spezifisch sind.

**Claims**

1. A method whereby Lp(a) and the various lipoproteins in a biological medium are separated by electrophoresis, **characterised in that** electrophoretic migration of lipoproteins is effected under conditions such that the electrophoresis gel and/or the said biological medium contains compounds capable of differentially modifying the electrophoretic mobility of Lp(a) relative to that of the other proteins, the compounds being chosen from among negatively charged molecules having a hydrophobic part, neutral surface-active agents, cations having a degree of oxidation of 2 or more and the cationic complexes $NH_4^+$, $(NH_4^+)_2 Fe^{2+}$ and $NH_4^+ Fe^{3+}$.

2. A method according to claim 1, **characterised in that** the said compound is adapted to separate the Lp(a) fraction from the VLDL and LDL fractions in a biological medium.

3.  A method according to claim 1 or 2, **characterised in that** the compound is adapted to separate the Lp(a) fraction from the VLDL and LDL fractions in a biological medium while keeping the HOL, LDL and VLDL separate.

4.  A method according to any of claims 1 to 3, **characterised in that** the cations or cationic complexes are those wherein the hydroxides, at pH from about 8 to about 9, are not or are only partly precipitated, so that there are at least $10^{-3}$ mols/l of non-precipitated cations or cationic complexes in the electrophoresis gel.

5.  A method according to any of claims 1 to 4, **characterised in that** the cations are chosen from among columns IIa, IIIa and IIIb in Mendeleev's Table.

6.  A method according to any of claims 1 to 4, **characterised in that** the cations are chosen from among $Mg^{2+}$, $Ca^{2+}$, $Ba^{2+}$, $Sr^{2+}$, $Mn^{2+}$, $Be^{2+}$, $Al^{3+}$, $La^{3+}$, $Ce^{3+}$, $In^{3+}$ and $y^{3+}$.

7.  A method according to any of claims 1 to 6, **characterised in that** the cation is $Mg^{++}$.

8.  A method according to any of claims 1 to 7, **characterised in that** the concentration of cations or cationic complexes in the electrophoresis gel is of the order of $10^{-3}$M to $10^{-1}$M.

9.  A method according to any of claims 1 to 8, **characterised in that** the concentration of cations or cationic complexes in the electrophoresis gel is of the order of $5 \times 10^{-3}$M to $3 \times 10^{-2}$M.

10. A method according to any of claims 1 to 3, **characterised in that** the compound capable of differentially modifying the electrophoretic mobility of Lp(a) relative to the other lipoproteins is a negatively-charged molecule having a hydrophobic part.

11. A method according to claim 10, **characterised in that** the negatively charged molecule having a hydrophobic part is either

    •   in the class of anionic surface-active agents and comprises either

        •   a straight or branched aliphatic chain of 3 to 10 carbon atoms, possibly comprising heteroatoms comprising an acid group e.g. sulphuric, sulphonic, carboxylic or phosphoric, and a condensed aliphatic chain containing at least 1 to 6 saturated rings of 5 to 6 carbon atoms or
        •   a straight or branched aliphatic chain of at least 8 carbon atoms, substituted if required by one or more aromatic rings or heterocyclic compounds, the chain or aromatic ring or rings or heterocyclic compound or compounds being substituted by an acid group such as carboxylic, sulphonic, sulphuric or phosphoric or
        •   is a negatively-charged aromatic derivative comprising at least two condensed aromatic rings or one aromatic ring condensed with an aromatic heterocyclic compound or two condensed aromatic heterocyclic compounds, at least one of the rings comprising an acid group such as carboxylic, sulphonic, sulphuric or phosphoric and the other ring not containing a group capable of destroying the hydrophobic character of the entire molecule.

12. A method according to claim 10 or 11, **characterised in that** the negatively charged molecule having a hydrophobic part is in the class of anionic surface-active agents and comprises a straight or branched aliphatic chain of 10 to 18 carbon atoms.

13. A method according to claim 11, **characterised in that** the aromatic derivative comprising two aromatic rings is naphthalene or the aromatic ring condensed with an aromatic heterocyclic compound is quinoleine.

14. A method according to claim 11, **characterised in that** the other ring does not have a polar charge.

15. A method according to any of claims 10 to 14, **characterised in that** the negatively charged molecule having a hydrophobic part is chosen from among the following compounds:

    -   sodium dodecyl sulphate,
    -   sodium dodecyl benzene sulphonate,

- sodium cholate,
- naphthalene 2-carboxylic acid,
- naphthalene 2-sulphonic acid,
- 1-sulphonic naphthalene 2-amino acid,
- 1-sulphonic naphthalene 4-hydroxy acid,
- 5-sulphonic naphthalene 1-amino acid,
- Noir Amido,
- Violet Acide 17,
- Bleu de Coomassie R250,
- Palatine fast black wan,
- 2-carboxylic naphthalene 1-hydroxy acid and
- 2-carboxylic naphthalene 3-hydroxy acid.

16. A method according to claim 10 or 11, **characterised in that** the negatively charged molecule having a hydrophobic part is 2-carboxylic naphthalene 1-hydroxy acid or 2-carboxylic naphthalene 3-hydroxy acid.

17. A method according to any of claims 1 to 3, **characterised in that** the compound capable of differentially modifying the electrophoretic mobility of Lp(a) relative to the other lipoproteins is a neutral surface-active agent (or neutral detergent).

18. A method according to claim 17, wherein the neutral surface-active agent is soluble in the aqueous phase.

19. A method according to claim 17 or 18, **characterised in that** the neutral surface-active agent is chosen from among Triton X100®, Triton X405®, Tween 20®, NP40® and BRIJ35®.

20. A method according to any of claims 11 to 19, **characterised in that** the concentration of anionic surface-active agents or neutral surface-active agents in the electrophoresis gel is of the order of $10^{-6}$ to $10^{-3}$M, or $10^{-4}$ to $10^{-1}$M if previously introduced into the biological medium.

21. A method according to claim 20, **characterised in that** the concentration of anionic or neutral surface-active agents in the electrophoresis gel is $10^{-4}$ to $10^{-3}$M.

22. A method according to claim 20, **characterised in that** the anionic or neutral surface-active agents are previously introduced into the biological medium and the concentration is of the order of $10^{-3}$ to $10^{-2}$M.

23. A method according to any of claims 11 to 16, **characterised in that** the concentration of negatively charged aromatic derivatives in the gel is about $10^{-3}$ to $10^{-1}$M, or about $10^{-2}$M to about 1M if they are previously introduced into the biological medium.

24. A method according to claim 23, **characterised in that** the concentration of negatively-charged aromatic derivatives in the gel is about $10^{-2}$ to $5.10^{-2}$ M.

25. A method according to claim 23, **characterised in that** the concentration of negatively-charged aromatic derivatives are previously introduced into the biological medium and the concentration is about $5.10^{-2}$ to about $5.10^{-1}$ M.

26. Application of the method according to any of claims 1 to 25 to determination of the atherogenic risk in man associated with the presence of Lp(a) in the organism.

27. A method of <u>in vitro</u> determination of the atherogenic risk associated with the presence of Lp(a), using a biological sample taken from man and containing:

   - a deposit of a suitable quantity of the biological sample in liquid form on an electrophoresis gel,
   - separation by electrophoresis of the Lp(a) and the various lipoproteins in the biological sample according to any of claims 1 to 25 and
   - detection of any Lp(a) present in the biological sample.

28. A method of <u>in vitro</u> determination of the atherogenic risk associated with the presence of Lp(a) according to claim 27, using a biological sample taken from man, in which the electrophoresis gel contains at least one compound capable of differentially modifying the electrophoretic mobility of Lp(a) relative to the other lipoproteins, as defined in any of claims 1 to 25.

**29.** A method of in vitro determination of the atherogenic risk associated with the presence of Lp(a) according to claim 27, using a biological sample taken from man and comprising:

- incubation of the biological sample with a solution of neutral surface-active agents or negatively charged molecules having a hydrophobic part,
- deposition of a suitable quantity of thus- treated biological sample on to a conventional gel for electrophoresis,
- an electrophoretic migration step, and
- detection of Lp(a) present in the biological sample.

**30.** A method according to claim 29, **characterised in that** the negatively-charged molecules having a hydrophobic part are defined in any of claims 11 to 16.

**31.** A method according to claim 29, **characterised in that** the neutral surface-active agents are defined in claim 18 or 19.

**32.** A method according to any of claims 27 to 31 **characterised in that** the biological sample taken from man is serum or plasma.

**33.** A method of determining the atherogenic risk associated with the presence of Lp(a) according to any of claims 27 to 32, **characterised in that** detection is effected by using a dye specific to lipoproteins or enzymatic reagents specific to lipids, or labelled antibodies directed against lipoproteins.

**34.** A method according to any of claims 27 to 33, **characterised in that** detection is made with Noir Soudan, Rouge Soudan or antibodies directed against Lp(a).

**35.** A method of determining the atherogenic risk associated with the presence of Lp(a) according to any of claims 27 to 34, **characterised in that** it comprises an additional step of dosage of the Lp(a).

**36.** A method of determining the atherogenic risk associated with the presence of Lp(a) according to claim 35, **characterised in that** the dosage of Lp(a) is carried out according to the method of LAURELL et al.

**37.** Use of the following compounds for electrophoretic separation of Lp(a)and of the various lipoproteins in a biological medium:

- an electrophoresis gel containing at least one compound capable of differentially modifying the electrophoretic mobility of Lp(a) relative to the other lipoproteins as defined in any of claims 1 to 25 and
- the buffer solution or solutions needed for electrophoretic migration of the lipoproteins on the gel and
- the reagents for detecting the lipoproteins including Lp(a) after migration on the gel.

**38.** Use for electrophoretic separation of Lp(a) according to claim 37, **characterised in that** the reagents for detecting the lipoproteins are chosen from among Noir Soudan dye, Rouge Soudan or enzymatic reagents specific to the lipids.

**39.** Use according to claim 37, also comprising:

- one or more solutions of the compounds, used either

  - for re-buffering the gel or
  - for adding to the biological sample.

**40.** Use according to claim 39, **characterised in that** the compounds are chosen from among those in claims 1, 11 or 18.

**41.** Use for electrophoretic separation of Lp(a)and of the various lipoproteins according to claim 39 or 40, **characterised in that** the reagents for detecting the lipoproteins are chosen from among Noir Soudan dye, Rouge Soudan or enzymatic reagents specific to the lipids.

**Figure 1A**          **Figure 1B**

HDL ——

VLDL + Lp (a) ——

LDL ——

1    2    3    4       1    2    3    4

**Figure 2A**          **Figure 2B**

HDL ——

Lp (a) ——

VLDL ——

LDL ——

1    2    3    4       1    2    3    4

**Figure 3**

HDL
Lp (a)
VLDL
LDL

1    2    3    4    5    6

**Figure 4**

HDL
VLDL
LDL
Lp (a)
Dépôts

1    2    3    4    5    6    7    8

**Figure 5A**          **Figure 5B**

1   2   3   4      1   2   3   4

**Figure 6**

HDL

VLDL + Lp (a)

LDL

Dépôt

HDL

VLDL

LDL

Lp (a)

Dépôt

⊖ ————————→ ⊕

prémigration